# EUROPEAN PATENT APPLICATION

(11) **EP 1 656 930 A1**
(43) Date of publication of application: **17.05.2006**
(21) Application number: 04405690.1
(22) Date of filing: 10.11.2004
(51) Int. Cl.: A61K 9/19, A61K 47/26, A61K 47/32, A61K 31/546

(54) **Stabilized freeze-dried formulation for cephalosporin derivatives**

(71) Applicant: Basilea Pharmaceutica AG, 4005 Basel (CH)
(72) Inventor: Heubes, Markus, Dr., 79650 Schopfheim (DE); Scigalla, Wilhelm, 79108 Freiburg (DE)
(74) Representative: Hoechst, Bruno Werner

(57) **Abstract**

The present invention relates to a freeze-dried formulation for cephalosporin derivatives having increased stability, a solution for obtaining and a method for preparing such a formulation, as well as the use of certain compounds for stabilizing cephalosporin derivatives in freeze-dried formulations. The compounds preferably used as stabilizers according to the invention are mannitol, trehalose, and PVP.

## Description

The present invention relates to a freeze-dried formulation for cephalosporin derivatives having increased stability, a solution for obtaining and a method for preparing such a formulation, as well as the use of certain compounds for stabilizing cephalosporin derivatives in freeze-dried formulations.

It is known that freeze-drying may have a considerable effect on the degradation of the pharmaceutically active ingredients in a formulation, as well as a strong impact on their stability in freeze-dried form. The variables which affect these parameters are mainly the pH, the quantity of salts present, the type and quantity of excipients in the formulation as well as the temperatures, pressure and time chosen for the freezing, sublimation and drying operations.

For the stabilization of the freeze-dried products, amino acids, and polyols are often used; but the literature, which is highly abundant on the subject, gives no information on the solution to the general problem of obtaining a stable pharmaceutical formulation.

More particularly, the literature teaches that the presence of an amino acid, of a polyol, for example mannitol, of a crystalline phase or of an amorphous phase may have, besides certain advantages, disadvantages which lead, in the case of freeze-dried products containing particularly sensitive active ingredients like for example cephalosporin derivatives, to relatively short shelf lives and/or low storage temperatures for these freeze-dried products.

The role of polyols and of amino acids has been studied separately in the case of the human growth hormone (hGH), but their synergistic effect is still poorly elucidated (Pikal M. J., Dellermann K. M., Roy M. L., Riggin M. N., The effects of formulation variables on the stability of freeze-dried Human Growth Hormone, Pharm. research., 1991, 8, No. 4, 427-436).

The advantages and disadvantages linked to the presence of amino acids and of mannitol are listed below.

Advantages linked to the presence of amino acids.

It has been demonstrated that the presence of glycine in a freeze-dried product induced crystallization of the molecules present in solution during the freezing stage of the freeze-drying (Korey D. J., Schwartz J. B., Effects of excipients on the crystallization of pharmaceutical compounds during lyophilization, J. Parenteral Sci. Tech., 1989, 43(2): 80-83). This crystallization of the active ingredient makes it possible to enhance its stability.

Alanine, in crystallized form, has the advantage of preventing the collapse of the freeze-dried product during sublimation and drying and or allowing the production of a freeze-dried product with a greater specific surface area and therefore allows a more rapid drying (Pikal M. J., Freeze-drying of proteins, Biopharm., 26-30 October 1990).

Disadvantages linked to the presence of amino acids.

The addition of an amino acid to a sugar or to a polyol in a solution to be freeze-dried generally has the effect of decreasing the glass transition temperature of the sugar (te Booy M. P. W. M., de Ruiter R. A., de Meere A. L. J., Evaluation of the physical stability of freeze-dried sucrose containing formulations by differential scanning calorimetry, Pharm. Research., 1992, 9, 109-114). Now, a decrease in the glass transition temperature is generally synonymous with a lower stability of a freeze-dried product (Franks F., Freeze-drying; from empiricism to predictability, Cryo-letters, 1990, 11, 93-110).

### Advantages linked to the presence of mannitol.

The presence of mannitol in the composition of a freeze-dried protein product is generally justified as bulking agent, that is to say that it makes it possible both to maintain the solid and rigid structure of the volume of the freeze-dried product corresponding to the volume of solution to be freeze-dried, but its presence also makes it possible to adjust the isotonicity of the reconstituted solution to be injected. When mannitol is the predominant excipient in the composition of a freeze-dried protein product, it is most often in crystalline form (Lyophilized formulations recombinant tumor necrosis factor, Hora M. S., Rana R. K., Smith F. W., Pharm. Res., 1992, 9 (1), 33-36).

### Disadvantages linked to the presence of mannitol.

It has been reported that the degree of hydrolysis of methylprednisolone sodium succinate, in freeze-dried form, was greater in the presence of mannitol than in the presence of lactose, and that this level increased with the quantity of mannitol present in the freeze-dried product. This has been explained by the fact that the crystallization of mannitol during freeze-drying changes the distribution of water in the matrix of the freeze-dried product. The increase in the quantity of water present in the microenvironment of the active ingredient resulting therefrom enhances the hydrolysis of the active ingredient and reduces its stability (The effect of bulking agent on the solid state stability of freeze dried methylprednisolone sodium succinate, Herman B. D., Sinclair B. D., Milton N., Nail S. L., Pharma. Res., 1994, 11 (10), 1467-1473).

The kinetics of degradation for various cephalosporin derivatives in aqueous solutions for various carbohydrates, polyhydric alcohols was shown to increase with increasing concentration of the hydroxyl compound concentration (The influence of carbohydrates and polyhydric alcohols on the stability of cephalosporins in aqueous solutions, Hans Bundgaard, Claus Larsen, Intl. Journal of Pharmaceutics, October 1983, 16, 3, 319-325).

Moreover, mannitol in freeze-drying is commonly used as carrier or constituting agent to result in a homogenous, stable cake with good appearance, it is not known as stabilizer for nonprotein compounds, in particular cephalosporin derivatives (see e.g. Handbook of Pharmaceutical Excipients, Rowe, R.C., Sheskey, P.J, Weller, P.J, Fourth Edition, PhP, London, 373-377.)

In conclusion, the scientific literature on the subject of the effect of excipients on the stabilization of pharmaceutical active ingredients gives contradictory information on their properties and furthermore does not make it possible to obtain some information on the subject of the relationships between the structure of a freeze-dried product and its stability. Likewise, the role of the polyols and of the amino acids, alone or in combination, is not described according to a set of generalizable properties, but has been observed with contradictory results according to the active principles studied and the quantities of excipients used.

Based on the above described state of the prior art, it is the object of the present invention to provide a freeze-dried formulation for cephalosporin derivatives showing an increased stability during manufacture of the formulation and/or during subsequent storage.

This object was solved by the freeze-dried formulation according to claim 1, a solution for obtaining a freeze-dried formulation according to claim 14, a method for preparing such a formulation according to claim 15, and the use of certain stabilizers according to claim 17.

According to the present invention, it was surprisingly found that certain substances have an unexpected stabilizing effect on freeze-dried formulations of cephalosporin derivatives.

Therefore, the present invention relates to a pharmaceutical formulation provided in the form of a freeze-dried product and containing at least one cephalosporin derivative as an active ingredient and at least one stabilizer selected from the group consisting of carbohydrates, polyhydric alcohols and polyvinyl pyrrolidone (PVP).

In this inventive formulation, the cephalosporin derivatives are stabilized at temperatures which may be as high as 25°C, or even higher, leading to an increased shelf life.

The stabilization by the inventive formulation of said cephalosporin derivatives includes the stabilization during manufacture of the product.

In particularly preferred embodiments, the stabilizer is selected from mannitol, trehalose, and PVP.

The active ingredient contained in the formulation according to the invention may be a single active ingredient or may be combined with another antibiotically active ingredient of protein or nonprotein nature.

In addition to carbohydrates, polyhydric alcohols and/or PVP, the formulation may further comprise one or more compounds selected from buffers, amino acids, acids or bases for adjusting the pH, surfactants, salts, preservatives, antioxidants, chelating agents.

While buffers and amino acids may lead to an additional stabilizing effect, the further components mentioned above are well-known pharmaceutically acceptable excipients often used in freeze-dried forms. Further customary additives known to a person skilled in the preparation of pharmaceutical formulations such as flavouring agents or dyes may be added as well.

Among the buffers which may be introduced into the formulation according to the present invention, there may be mentioned in particular citrate, tri(hydroxymethyl)aminomethane, maleate, succinate, tartrate, carbonate, and hydrogene carbonate buffers, as well as mono-acidic buffers like lactate, glycine, or acetate buffer systems. It is being understood that the acids and bases composing said buffers may also be introduced alone, including hydrates, as well as any combinations thereof.

Among the surfactants which may be introduced into the formulation according to the present invention, there may be mentioned polysorbates, poloxamers, tyloxapol, lecithins.

Among the salts which may be introduced into the formulation according to the present invention, there may be mentioned in particular the sodium salts such as ededate (tetrasodium EDTA), chloride, docusate (sodium 1,4-bis(2-ethylhexyl)sulphosuccinate), bicarbonate, glutamate, potassium acetate, dipotassium carbonate and magnesium stearate.

Among the preservatives which may be introduced into the formulation according to the present invention, there may be mentioned in particular methyl and propyl para-hydroxybenzoate, benzethonium chloride, sodium mercurothiolate, phenyl-mercuric nitrate, benzyl alcohol, phenol and metacresol.

The formulations according to the present invention may be either reconstituted in liquid form by addition of an adequate solvent or reconstitution solution for its administration via the parenteral, intra-muscular or oral route, or directly administered via the oral route, to man or to animals. In addition, the liquid or dry forms may be administered by inhalation.

The cephalosporin derivatives in the present invention include all pharmaceutically acceptable salts and polymorphs as well as hydrates. Furthermore, the term cephalosporin derivative is also meant to include drugs as well as prodrugs.

In a preferred embodiment, the present formulation comprises a cephalosporin derivative of the following general formula I 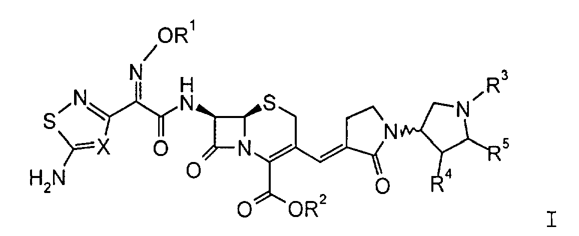 wherein
- R¹: is hydrogen, C₁₋₆-alkyl, optionally substituted by fluoro, or C₃₋₆-cycloalkyl;
- R²: is hydrogen or a group selected from -CH₂C(=CHR)-COOR, -CH₂OCOR, -CH (R) OCOR, -CH (R) OCOOR, -CH (OCOR) OCOR, -CH₂COCH₂OCOR and 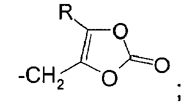
- R³: is hydrogen or group selected from -CH₂C(=CH₂)-COOR,
-COOCH₂C(=CHR)-COOR, -COOCH₂OCOR,
-COOCH(R)OCOR, -COOCH(R)OCOOR, -COOCH (OCOR) OCOR, -COOCH₂COCH₂OCOR, and 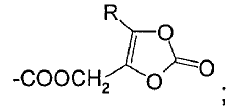 with the proviso that one of R² and R³ is hydrogen and the other of R² and R³ is different from hydrogen,
- R: is hydrogen or C₁₋₆-alkyl;
- R⁴: is hydrogen or hydroxy,
- R⁵: is hydrogen or ω-hydroxyalkyl; and
- X: is CH or N,
as well as pharmaceutically acceptable salts and polymorphs of said compounds and hydrates of the compounds of formula I and of their salts.

Compounds according to the above formula I which are known to suffer from stability problems in freeze-dried formulations are for example described in European patent no. EP 1 087 980 B1.

A particularly preferred example of the compounds of formula I is (6R,7R)-7-[(Z)-2-(Amino-[1,2,4]thiadiazol-3-yl)-2-hydroxyimino-acetylamino]-3-[(E)-(3'R,5'R)-5'-hydroxymethyl-1'-(5-methyl-2-oxo-[1,3]dioxol-4-ylmethyloxycarbonyl-2-oxo-[1,3']bipyrrolidinyl-3-ylidenemethyl]-8-oxo-5-thia-1-azabicyclo[4.2.0]oct-2-ene-2-carboxylic acid wherein R¹, R², R⁴ and R⁵ are all hydrogen, R³ is 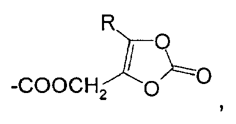 and R is methyl. In the following, this compound is referred to as BAL 5788.

The formulations according to the present invention may be obtained by freeze-drying an aqueous solution comprising at least one cephalosporin derivative as an active ingredient, and at least one stabilizer selected from the group consisting of carbohydrates, polyhydric alcohols and PVP. In particular the stabilizer is selected from mannitol, trehalose, and PVP.

The stabilizer is preferably contained in the solution at a concentration within the range of 5 to 80% by weight. In particular, mannitol, trehalose, and PVP are used at a concentration of 2 to 40% by weight. Especially preferred are concentrations ranging from 10 to 25% by weight. Percent by weight in this application always refers to the dry weight.

The solution may also contain a pharmaceutically acceptable buffer for further stabilization and/or for adjusting the pH such as citrate, tartrate, carbonate, hydrogen carbonate, lactate, glycine, acetate or succinate buffers.

The preferred pH range is from 2.0 to 6.5, wherein 4.0 to 5.0 is particularly preferred.

The quantity of the active ingredient present is limited by its solubility in the aqueous solution. The formulations of the invention indeed result from the freeze-drying of aqueous solutions in which the active ingredient is perfectly dissolved.

A particularly preferred solution for forming a formulation according to the present invention comprises a cephalosporin derivative according to formula I, mannitol as a stabilizer and an aqueous citrate buffer.

The solutions to be freeze-dried are, for example, prepared in the following manner:

The desired quantities of active ingredient, stabilizer and buffer, and optionally further additives like for example preservatives are added, at the appropriate dissolution temperature, to the quantity of water for injection or of solubilizing agent necessary for their solubilization until complete dissolution is obtained. The solutions obtained are filtered in a sterile medium and distributed into containers, preferably vials or capsules.

The freeze-drying of the solutions may then be carried out as follows:

The solution follows a cycle comprising freezing, then sublimation and drying adapted to the volume to be freeze-dried and to the container containing the solution.

The sublimation and drying times, temperatures and pressures are adjusted according to the volumes of solution to be freeze-dried and the residual water content desired in the freeze-dried product.

The present invention will now be described by way of specific examples which are however not intended to limit the scope of the present invention.

The following solutions were prepared, lyophilised, and the resulting formulations were tested with regard to their stability:

### Preparation of Solution A (with mannitol):

Solution A was prepared by dissolution of 192.0 g BAL 5788 (synthesized as described in EP-A-1 087 980) and 34.38 g mannitol (obtained from Roquette America, Inc.) in a sodium hydroxide/citric acid buffer system with a pH between 4.2 - 4.8 (prepared by dissolving 2.18 g citric acid monohydrate in WFI (water for injection) and pH adaptation with sodium hydroxide) to result the final weight of 1389.5 g. The solution was filtered and filled into vials.

### Lyophilisation of Solution A:

Solution A was freeze-dried according to the process summarized in the following table to obtain formulation A.

| Freeze-drying stage | Shelf Temperature [°C] | Pressure [µbar] |
|---|---|---|
| Freezing | Approximately -48 | Ambient |
| Primary drying | Ramping from -40 to -30 | Approximately ≤ 70 |
| Secondary drying | Ramping from -30 to 30 | Approximately ≤ 70 |

### Preparation of a Reference Solution B (without mannitol):

The reference solution B was prepared in the same way as solution A by dissolution of an equal amount of BAL 5788, in the same sodium hydroxide/citric acid buffer system in WFI with the only difference that no mannitol was added. The solution was filtered and filled into vials.

The reference solution was freeze-dried as outlined above obtaining formulation B.

The freeze dried products resulting from Solution A and Solution B were analytically characterized and a stability testing program was initiated covering different temperatures.

### Compositions (nominal) of Formulation A and Formulation B

### Composition of Formulation A (per vial):

| Compound | Weight [mg] |
|---|---|
| Ba15788 | 999.8 |
| Citric Acid Monohydrate | 15.9 |
| Mannitol | 179.1 |
| NaOH qs to | 4.2 - 4.8 pH |
| WFI | ≤ 3% |

### Composition of Formulation B (per vial):

| Compound | Weight [mg] |
|---|---|
| Ba15788 | 999.8 |
| Citric Acid Monohydrate | 15.9 |
| NaOH qs to | 4.2 - 4.8 pH |
| WFI | ≤ 3% |

### Results:

The amount of degradation products formed during manufacture of Formulation A (with mannitol) was approximately 9% lower compared to Formulation B (without mannitol).

The amount of degradation products formed during storage of the freeze-dried product (at e.g. 5°C, after 12 months) obtained from Solution A (with mannitol) was approximately 10% lower compared to the amount formed during storage of the freeze-dried product resulting from Solution B (without mannitol).

Further formulations were prepared in the same way with trehalose or PVP, and also with other buffer systems.

## Claims

1. Freeze-dried formulation for cephalosporin derivatives, which is pharmaceutically acceptable, comprising at least one cephalosporin derivative as an active ingredient and at least one stabilizer selected from the group consisting of carbohydrates, polyhydric alcohols and polyvinyl pyrrolidone (PVP).

2. Freeze-dried formulation according to claim 1, wherein the carbohydrate is trehalose.

3. Freeze-dried formulation according to claim 1, wherein the polyhydric alcohol is mannitol.

4. Freeze-dried formulation according to any of claims 1 to 3, further comprising an additional antibiotically active ingredient.

5. Freeze-dried formulation according to any of claims 1 to 4, further comprising one or more compounds selected from buffers, amino acids, acids or bases for adjusting the pH, surfactants, salts, preservatives, antioxidants, chelating agents.

6. Freeze-dried formulation according to any of claims 1 to 5 for reconstitution of a solution for its administration via the parenteral route, the intramuscular route, the oral route or via inhalation.

7. Freeze-dried formulation according to any of claims 1 to 6, wherein the cephalosporin is a compound of the following general formula I 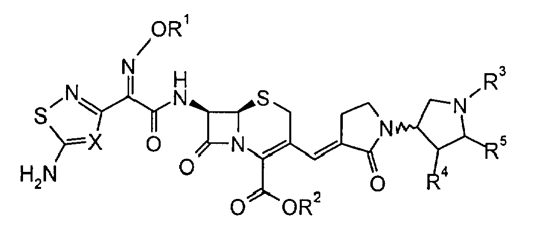 wherein
R¹ is hydrogen, C₁₋₆-alkyl, optionally substituted by fluoro, or C₃₋₆-cycloalkyl;
R² is hydrogen or a group selected from -CH₂C(=CHR)-COOR, -CH₂OCOR, -CH(R)OCOR, -CH (R) OCOOR, -CH (OCOR) OCOR, -CH₂COCH₂OCOR and 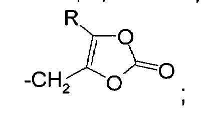
R³ is hydrogen or group selected from -CH₂C(=CH₂)-COOR,
-COOCH₂C(=CHR)-COOR, -COOCH₂OCOR, -COOCH(R)OCOR, -COOCH(R)OCOOR, -COOCH(OCOR)OCOR, -COOCH₂COCH₂OCOR, and 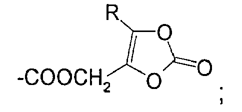 with the proviso that one of R² and R³ is hydrogen and the other of R² and R³ is different from hydrogen,
R R is hydrogen or C₁₋₆-alkyl;
R⁴ is hydrogen or hydroxy,
R⁵ is hydrogen or ω-hydroxyalkyl; and
X is CH or N,
as well as pharmaceutically acceptable salts and polymorphs of said compounds and hydrates of the compounds of formula I and of their salts.

8. Formulation according to any of claims 1 to 7 obtained after freeze-drying a solution comprising at least one cephalosporin as an active ingredient, at least one stabilizer selected from the group consisting of carbohydrates, polyhydric alcohols and PVP, and an aqueous solution.

9. Formulation according to claim 8, wherein the stabilizer is mannitol, trehalose or PVP.

10. Formulation according to claim 8 or 9, wherein the aqueous solution is a buffer solution, preferably a citrate, tartrate, carbonate, hydrogen carbonate, succinate, glycine, lactate or acetate buffer solution.

11. Formulation according to any of claims 8 to 10, wherein the aqueous solution is a mono-acidic buffer solution e.g. an acetate, glycine or lactate buffer solution.

12. Formulation according to any of claims 8 to 11, wherein the concentration of the stabilizer in the solution is in the range of 5 - 80% by weight.

13. Formulation according to any of claims 8 to 12, wherein the pH of the solution is in the range of 2.0 - 6.5, in particular in the range of 4.0 - 5.0.

14. Solution for obtaining a freeze-dried formulation, comprising at least one cephalosporin derivative as an active ingredient, at least one stabilizer selected from the group consisting of mannitol, trehalose and PVP in an aqueous solution.

15. Method for preparing a stabilized freeze-dried pharmaceutically acceptable formulation for cephalosporin derivatives, comprising the steps of
(a) adding at least one stabilizer selected from carbohydrates, polyhydric alcohols, and PVP to an aqueous solution of a cephalosporin derivative; and
(b) freeze-drying the above solution.

16. Method according to claim 15, **characterized in that** mannitol, trehalose or PVP is added as the stabilizer.

17. Use of a compound selected from the group consisting of carbohydrates, polyhydric alcohols and PVP for stabilizing cephalosporin derivatives in freeze-dried formulations.

18. Use according to claim 17, **characterized in that** the compound is mannitol, trehalose or PVP.
